# EUROPEAN PATENT SPECIFICATION

(11) **EP 4 384 092 B1**
(45) Date of publication and mention of the grant of the patent: **23.09.2026**
(21) Application number: 22768928.8
(22) Date of filing: 09.08.2022
(51) Int. Cl.: A61B 17/29

(54) **MEDICAL DEVICE WITH DISTAL ROTATION**
MEDIZINISCHE VORRICHTUNG MIT DISTALER ROTATION
DISPOSITIF MÉDICAL À ROTATION DISTALE

(30) Priority: 12.08.2021 CN 202110924224
(43) Date of publication of application: 19.06.2024
(73) Proprietor: Boston Scientific Scimed, Inc., Maple Grove, MN 55311 (US)
(72) Inventor: HUA, Minfeng, Shanghai 200052 (CN); PEI, Xinzhe, Shanghai 200237 (CN); REN, Hongbin, Tangshan Hebei (CN); XIA, Yin, Shanghai 200120 (CN); YING, Jizhe, Shanghai 201114 (CN); ZHANG, Cheng, Ann Arbor, MI 48105 (US); CAI, Longsheng, Shanghai 201600 (CN)
(74) Representative: Vossius & Partner Patentanwälte Rechtsanwälte mbB
(86) International application number: PCT/IB2022/000448
(87) International publication number: WO 2023/017313

(56) References cited:
- US-A- 5 439 478
- US-A1- 2004 220 565
- US-A1- 2008 147 113

## Description

### TECHNICAL FIELD

Various embodiments of this disclosure relate generally to medical devices having a rotatable end effector. Examples of the disclosure relate to ergonomic handles that control the rotation of the end effector.

### BACKGROUND

In some medical procedures, a physician may require precise manipulation of an end effector. For example, during endoscopic surgeries, a physician may use accessory devices, such as forceps, baskets, nets, etc., to access and treat a site within a patient. The physician or a technician may introduce an accessory device into a working channel of a scope, such as an endoscope, to reach a treatment site within the patient. Once the site is reached, the physician may proceed to provide treatment to the desired site. Currently, devices often do not have the capability to rotate the end effector independently from the shaft or handle in a precise manner. Imprecise manipulation of the end effector is disadvantageous during such procedures, as it can lead to increased procedure times and difficulty accessing a treatment site or delivering treatment.

US 5,439,478 A discloses a steerable flexible microsurgical instrument with a rotatable clevis. The instrument includes a flexible coil, a flexible pull wire extending through the coil, a clevis which is rotatably coupled to the distal end of the coil, a handle coupled to the proximal end of the coil, and end effectors coupled to the clevis and the pull wire. The clevis is coupled to the distal end of the flexible coil by a bushing which allows axial rotation of the clevis relative to the coil. The pull wire is formed from torsionally obdurate material and is coupled at its distal end to the end effector and at its proximal end to a movable part of the handle. The handle is provided with mechanism for rotating the pull wire relative to the coil and a mechanism for translating the pull wire relative to the coil. Translation of the pull wire relative to the coil moves the end effector relative to the clevis and rotation of the pull wire relative to the coil rotates the clevis and end effector relative to the coil.

US 2008/147113 A1 and US 2004/220565 A1 disclose further surgical instruments.

This disclosure is directed to overcoming one or more of these above-referenced challenges or other challenges in the art.

### SUMMARY

Claim 1 defines the invention and dependent claims disclose embodiments. No surgical methods are claimed per se. Aspects of the disclosure relate to, among other things, devices and methods to enable actuation and rotation of a distal end effector to enable treatment at a target tissue site within a subject (e.g., patient). Each of the aspects disclosed herein may include one or more of the features described in connection with any of the other disclosed aspects.

The invention is defined by a distal end assembly of a medical device according to the independent claim. Preferred embodiments are defined in the dependent claims. The bushing of the distal end assembly may be fixed to the clevis. In other embodiments, the bushing may be within the clevis. In some embodiments, the end effector may comprise two jaws.

The distal end assembly may be configured such that translation of the distal actuator in a first direction opens the end effector and translation of the distal actuator in a second direction closes the end effector. The end effector may also or alternatively be pivotably connected to the distal actuator by one or more pins and one or more links. In some embodiments, the clevis is rotatable relative to the shaft connector. Rotation of the distal actuator may cause rotation of the clevis and the end effector. A should of the shaft connector and a flange of the bushing may bushing inhibit axial movement of the distal end assembly. Additionally, in some embodiments, the distal actuator translates within the bushing. The end effector may be pivotably coupled to the distal actuator by one or more wires.

In other embodiments, an annular gap is defined between the bushing and the shaft connector and/or an annular gap is defined between the distal actuator and the bushing. Additionally or alternatively, the shaft connector may define a lumen extending therein and an opening at a distal end, the bushing extending within the lumen and out of the opening. The bushing defines a lumen therethrough, and the distal actuator extends through the lumen.

According to other aspects of the disclosure, a medical device may comprise a handle having at least one actuator; a shaft having a proximal end and a distal end, the proximal end connected to the handle; a distal assembly connected to the distal end of the shaft, the distal assembly, and wire coupled to the at least one actuator and to a shaft connector. The distal assembly may include an end effector, a distal actuator, wherein translation of the distal actuator actuates the end effector, a bushing, and the shaft connector connected to the distal end of the shaft. The bushing may be within the shaft connector and rotatable relative to the shaft connector. Operation of the at least one actuator translates the wire to actuate the end effector and rotates the wire to rotate the end effector relative to the shaft.

In some embodiments, the at least one actuator comprises a first actuator and a second actuator, wherein first actuator is a knob rotatable relative to a stationary portion of the handle, wherein rotation of the knob rotates the end effector; and wherein the second actuator is an assembly slidably connected to the stationary portion of the handle such that translation of the second actuator in a first direction actuates the end effector and translation of the second actuator in a second direction different from the first direction actuates the end effector. The handle may comprise an electrical connector, and wherein the electrical connector is moveably coupled to the wire, and activation of the electrical connector electrically activates the end effector. A shoulder of the shaft connector and a flange of the bushing inhibit axial movement of the distal end assembly relative to the shaft.

Aspects of this disclosure also include exemplary methods of operating a medical device. These methods include positioning the medical device inside a body lumen, wherein the medical device includes a handle having at least a first actuator and a second actuator, a shaft having a proximal end and a distal end, the proximal end being connected to the handle, and a distal assembly connected to the distal end of the shaft, the distal assembly including an end effector; and rotating the end effector relative to the shaft with the first actuator, and actuating the end effector with the second actuator.

Additional objects and advantages of the disclosed embodiments will be set forth in part in the description that follows, and in part will be apparent from the description, or may be learned by practice of the disclosed embodiments. The objects and advantages of the disclosed embodiments will be realized and attained by means of the elements and combinations particularly pointed out in the is disclosure. The scope of the invention is defined by the appended claims.

It may be understood that both the foregoing general description and the following detailed description are exemplary and explanatory only and are not restrictive of the disclosure.

### BRIEF DESCRIPTION OF THE DRAWINGS

The accompanying drawings, which are incorporated herein and constitute a part of this specification, illustrate exemplary aspects of the disclosure and, together with the description, explain the principles of the disclosure.
FIG. 1 is a side view of a medical device, according to aspects of this disclosure;
FIG. 2 is a cross-sectional view of an exemplary handle of the medical device of FIG. 1, according to aspects of this disclosure;
FIG. 2A is an exploded view of portions of the handle of FIG. 2, according to aspects of this disclosure;
FIGs. 3A and 3B are side cross-sectional views of an exemplary end effector assembly in the open state (FIG. 3A) and in the closed state (FIG. 3B), according to the invention; and
FIG. 4 is a perspective view of an alternate embodiment of an end effector assembly, according to aspects of this disclosure.

### DETAILED DESCRIPTION

Aspects of the disclosure include devices and exemplary methods to enable and rotation of a distal end effector of a medical device, to enable treatment at a target tissue site within a subject (e.g., patient). In embodiments, the handle is configured so that a user may control the actuation and rotation of the distal end effector. The ability to manipulate (including rotate) the end effector in a precise manner enables a user to grasp a target more efficiently, thereby reducing procedure time, more precise grasping of tissue, and a decreased volume of blood loss, all of which may also result in better patient outcomes.

The medical device may be introduced into the body without a delivery device or via a delivery device. The delivery device may be a catheter, a scope (endoscope, bronchoscope, colonoscope, duodenoscope, etc.), a tube, a sheath, or other like device, inserted into a body cavity or lumen, for example the GI tract, via a natural orifice. The orifice can be, for example, the nose, mouth, or anus, and the placement can be in any portion of the GI tract, including the esophagus, stomach, duodenum, large intestine, or small intestine. Delivery and placement also can be in other body lumens or organs reachable via the GI tract, any natural opening, any other body tract, or any bodily incision.

Reference will now be made in detail to aspects of the disclosure, examples of which are illustrated in the accompanying drawings. Wherever possible, the same or similar reference numbers will be used through the drawings to refer to the same or like parts. The term "distal" refers to a portion farthest away from a user when introducing a device into a patient. By contrast, the term "proximal" refers to a portion closest to the user when placing the device into the subject. As used herein, the terms "comprises," "comprising," or any other variation thereof, are intended to cover a non-exclusive inclusion, such that a process, method, article, or apparatus that comprises a list of elements does not necessarily include only those elements but may include other elements not expressly listed or inherent to such process, method, article, or apparatus. The term "exemplary" is used in the sense of "example," rather than "ideal." As used herein, the terms "about," "substantially," and "approximately," indicate a range of values within +/- 10% of a stated value.

Examples of the disclosure may relate to devices and methods for performing various medical procedures and/or treating portions of the large intestine (colon), small intestine, cecum, esophagus, any other portion of the gastrointestinal tract, and/or any other suitable patient anatomy (collectively referred to herein as a "target treatment site"). Various examples described herein include single-use or disposable medical devices. Any structures of the medical devices described herein can be made of biocompatible materials, including biocompatible polymers, rubbers, plastics, and the like.

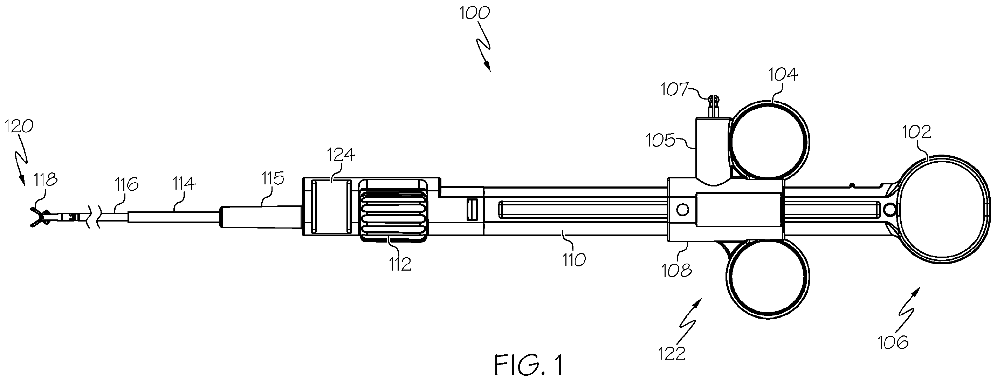

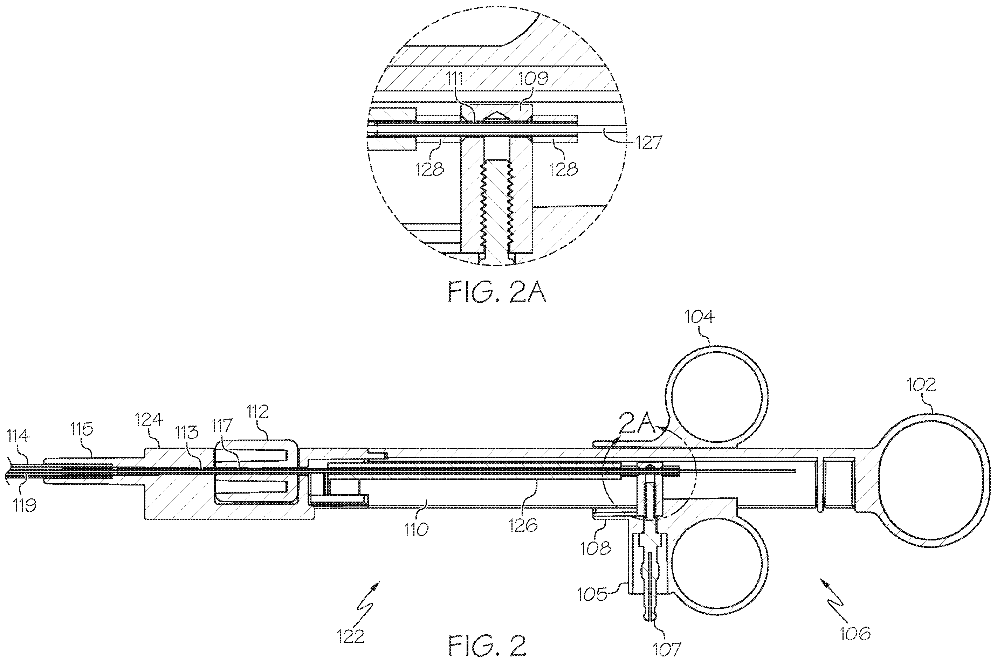

FIG. 1 is a general depiction of a medical device 100 in accordance with examples of this disclosure. Medical device 100 includes a proximal end 106 and a distal end 120. A handle assembly 122 is located at or proximal to proximal end 106 and includes one or more actuators 108, 112. FIG. 2 shows a cross-sectional view of handle assembly 122, flipped over relative to its position in FIG. 1. Actuator 108 may be located at or near a proximal end of handle assembly 122. Actuator 108 controls the actuation (e.g. opening and closing) of end effectors of medical device 100 and provides electrical current to the end effectors, as will be described. Actuator 108 may include one or more finger slot(s) 104 extending outwardly from actuator 108. The one or more finger slot(s) 104 may be located at or near a proximal end of actuator 108. Alternatively, the one or more finger slot(s) 104 may be located at or near a distal end of actuator 108. The one or more finger slot(s) 104 may be any suitable shape and size to enable a user to insert a finger through the slot. For example, the one or more finger slot(s) 104 may be circular (as shown), triangular, rectangular, cylindrical or any shape commonly known in the art. Actuator 108 may also include an electrical insert 107 within a cylindrical protrusion 105, both of which extend outwardly from a central portion of actuator 108. The electrical insert 107 and cylindrical protrusion 105 may be located at or near a distal end of actuator 108. Alternatively, the electrical insert 107 and cylindrical protrusion 105 may be located at or near a proximal end of actuator 108.

Actuator 112 may be located at or near a distal end of handle 122. Actuator 112 may include a knob, trigger, button, switch, pneumatic control, or any other actuator known in the art and may control the rotation of a distal end of medical device 100. Actuator 112, its interaction with other components of device 100, and its use are described further herein.

Handle 122 may further include a handle piece 110. Handle piece 110 may include a track on which actuator 108 is moveably coupled to such that actuator 108 may translate in a first direction, towards a distal end of handle 122, and in a second direction, towards a proximal end of handle 122. For example, handle piece 110 may include a central longitudinal slot through which a portion of actuator 108 extends. Actuator 108 translates longitudinally within that slot in the first and second directions. The movement of actuator 108 in a first direction and in a second direction results in the actuation or de-actuation of a distal end effector assembly 118 by means of, for example, a connection of an elongate member, such as a cable 126 (shown in FIG. 2), between the actuator 108 and the distal components. Cable 126 may include an electrically conductive wire covered by an insulative sheath 119. The connections of cable 126 to various components are described further herein.

Handle piece 110 includes a finger slot 102 located at or near a proximal end of handle piece 110. Finger slot 102 may be shaped similarly or differently as compared to the one or more finger slot(s) 104. For example, the one or more finger slot(s) 104 may be circular, and the finger slot 102 may be circular, ovular, or rectangular.

Handle 122 may also include a handle piece 124. Handle piece 124 may be fixedly coupled to a distal end of handle piece 110 or may be integrally formed with handle piece 110. Handle piece 124 may have a recess that accepts the actuator 112 and related mechanisms to facilitate the rotation of a distal end of medical device 100, to be described further herein. A cylindrical protrusion 115 extends from a distal end of handle piece 124. The protrusion 115 may provide structural support to a strain relief 114 and/or a shaft 116. Strain relief 114 may extend into and terminate within protrusion 115, as shown in FIG. 2. The termination of strain relief 114 may be accomplished by means of glue, overmold, press-fit, or any other means commonly used in the art. Additionally, shaft 116 may extend through strain relief 114 and terminate within protrusion 115 or handle piece 124. Similar to strain relief 114, the termination of shaft 116 within strain relief 114 or protrusion 115 may be accomplished by means of glue, overmold, press-fit, or any other means commonly used in the art.

Still referring to FIG. 1, a shaft 116 of device 100 extends from a distal end of handle 122 to the distal end 120 of device 100. Distal end 120 includes a distal end effector assembly 118, to be described further therein. Shaft 116 may be a tube having sufficient length to access sites within the body. Additionally, shaft 116 may have sufficient flexibility to traverse tortuous anatomy. Shaft 116 can be made of flexible materials, rigid materials, or any combination thereof.

FIG. 2 shows a cross-section of handle 122, showing components that are interior of handle pieces 110 and 124. Electrical insert 107 extends through protrusion 105 and into actuator 108. Electrical insert 107 is coupled, for example via a screw connection, to an electrically conductive electrical connector 109. Electrical connector 109 includes a thru-hole 111 that extends in a direction of the longitudinal axis of handle 122. The proximal portion of the electrically conductive wire 127 of cable 126 (exposed from its insulative sheath 119 at its proximal portion) may extend through thru-hole 111. Between the electrical connector 109 and the wire 127, there is a small gap to enable axial rotation of cable 126 (and its wire 127) relative to electrical connector 109. During this rotation, electrical contact is maintained between the cable 126, its wire 127, and the electrical connector 109. At least one or more cannula(s) 128 may be fixedly coupled to the outer diameter of wire 127. The one or more cannula(s) 128 may abut a distal end and/or a proximal end of the electrical connector 109, thereby preventing axial movement of wire 127 (and its cable 126) relative to the electrical connector 109. The electrical connector 109 provides an electrical interface between electrical insert 107 and the one or more cannulas 128 and wire 127 such that, when the electrical insert 107 is energized or activated (receives electrical current from a connected current source), the one or more cannula(s) 128 and wire 127 are also energized or activated. The connection between these components may provide the electrical current to the distal end effector assembly 118 of medical device 100.

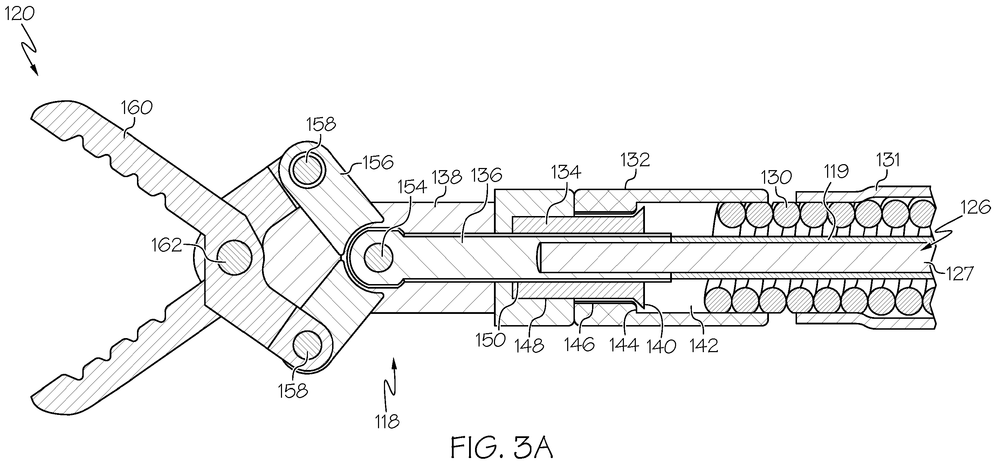

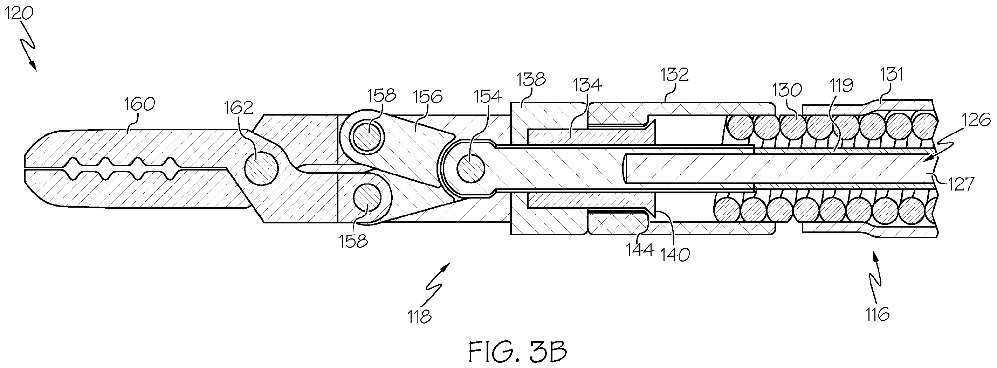

Actuator 112 may be nested in and moveably coupled to handle piece 124 in a manner permitting axial rotation of actuator 112 relative to handle piece 124 but limiting axial movement of actuator 112 relative to handle piece 124. A thru-hole 117 extends axially from a proximal end of actuator 112 to a distal end of actuator 112 through which cable 126 extends. The thru-hole 117 may be rectangular in shape and receive a same or similarly shaped tube 113 coupled to the outside of cable 126. Tube 113 may be coupled to cable 126 by means of a crimp, glue, or other means commonly known in the art. The configuration/connections between cable 126, thru-hole 117, tube 113, and actuator 112 enables rotation of end effector assembly 118 (shown in FIG. 1), when actuator 112 is rotated. For example, when actuator 112 is rotated clockwise, tube 113 and cable 126 rotate accordingly, thereby subsequently rotating an end effector assembly 118 coupled to a distal end of cable 126. Similarly, when actuator 112 is rotated counterclockwise, tube 113 and cable 126 rotate accordingly, thereby subsequently rotating an end effector assembly 118 coupled to a distal end of cable 126. As mentioned above, cable 126 may have an insulative sheath 119, extending from a distal end of cable 126 (see FIGs. 3A and 3B) to a proximal end. Insulative sheath 119 may terminate within tube 113 or a location distal to actuator 112 (e.g. within shaft 116), to expose wire 127 and permit the connections of wire 127 to other components within handle 122.

FIG. 3A is a cross-sectional view of an exemplary distal end effector assembly 118. End effector assembly 118 can include a variety of components, including tools and parts to connect the tools to other parts of medical device 100 and permit the various functions of those tools. Exemplary tools include, but are not limited to, a tissue grasper (such as an electrocautery/coagulation grasper as shown in the Figures), a knife, biopsy forceps, scissors, a retrieval device (such as a net or a basket), an electrocautery tool, etc. The electrocautery/coagulation grasper, shown in the Figures, enables a user to grasp tissue (particularly bleeding tissue, for example) and deliver energy for procedures in which coagulation or hemostasis is desirable to reduce bleeding. During such procedures and when otherwise necessary, the device 100 may be coupled to a generator (not shown) to provide monopolar or bipolar energy to the distal end effector assembly 118. As mentioned above, handle actuators (e.g. actuators 108, 112) and related mechanisms can control the rotation and actuation (e.g., open/close movement) of end effector assembly 118. For example, a connection between the actuators and the distal components, such as provided by cable 126, transmit the action of the actuators 108, 112 to the respective functionality at the distal end effector 118, the mechanisms of which are described in further detail below.

A distal end of shaft 116 may be fixedly coupled to end effector assembly 118 by a variety of means. For example, a coil 130 of shaft 116 may extend past a distal-most end of a covering 131 of shaft 116. A distal end of the coil 130 may be fixedly couple to a shaft connector 132 by an adhesive, a press-fit, ultrasonic welding, laser welding, or any other means commonly known in the art.

At least a portion of cable 126, and particularly wire 127, may extend past a distal-most end of shaft 116 and may be fixedly coupled to a distal actuator 136. Wire 127 may be fixedly coupled to distal actuator 136 by a variety of means, including adhesives, a press-fit, ultrasonic welding, laser welding, or any other means commonly known in the art. Alternatively, distal actuator 136 may extend into coil 130 such that wire 127 terminates within a distal portion of shaft 116. A distal end of distal actuator 136 may be coupled to one or more link(s) 156 by means of a pin 154, permitting pivoting of links 156 relative to distal actuator 136. Further, links 156 may be coupled to a pair of jaws 160 by means of one or more pin(s) 158, permitting pivoting of each jaw 160 relative to its associated link 156. A pin 162 may be fixedly coupled to a clevis 138 and moveably coupled to jaws 160 such that jaws 160 rotate about pin 162. The combination of and connections between the aforementioned components is such that axial translation of cable 126 towards distal end 120 may actuate (e.g. open) jaws 160. Alternatively, axial translation of cable 126 away from distal end 120 may de-actuate (e.g. close) jaws 160 (as shown in FIG. 3B).

To rotate the distal end effector assembly 118, cable 126 may be rotated by actuator 112, which is coupled to a proximal portion of wire 127, as shown in FIG. 2. A distal end of cable 126 is fixedly coupled to a proximal end of distal actuator 136, as described above. Distal actuator 136 may extend through an internal bushing 134 such that there is an annular gap between the distal actuator 136 and the bushing 134. Internal bushing 134 is a generally cylindrical component having an axially extending thru-hole. Internal bushing 134 extends within shaft connector 132 and distally of shaft connector 132 through a distal opening of shaft connector 132. A small annular gap exists between an outer surface of internal bushing 134 and an inner surface of shaft connector 132. Internal bushing 132 is rotatable relative to and within shaft connector 132. A radially outward extending flange 140 on a proximal end of internal bushing 134 abuts an internal shoulder 144 of shaft connector 132. Flange 140's interaction with shoulder 144 permits rotation of the internal bushing 134 relative to shaft connector 132, while preventing distal movement of internal bushing past the distal end of shaft connector 132.

Further, internal bushing 134 may be inserted into slot 148 and fixedly coupled to a proximal portion of the clevis 138. Internal bushing 134 may be coupled to clevis 138 by means of adhesives, press-fit, ultrasonic welding, laser welding, or other means commonly known in the art. During assembly, the internal bushing 134 may be inserted into a thru-hole 146 of the shaft connector 132. The internal bushing 134 may then be subjected to a secondary manufacturing step such that flange 140 is created. However, in either embodiment, though the proximal end of clevis 138 may abut or touch the distal end of shaft connector 132, the proximal end of clevis 138 is not coupled to the distal end of shaft connector 132, thereby enabling rotational movement of the end effector assembly 118. A gap 142 may be between a proximal face of internal bushing 134 and a distal end of coil 130 to account for any tolerance differences between these components that may arise during the manufacturing process.

Internal bushing 134 is moveably coupled to distal actuator 136 by matching of a similarly shaped thru-hole 150 of the internal bushing 134 and distal actuator 136. For example, the thru-hole 150 of internal bushing 134 may be rectangular and receive a rectangular-shaped distal actuator 136. Alternatively, the thru-hole 150 of internal bushing 134 may be ovular to receive a similarly shaped actuator 136. In either embodiment, when cable 126 is rotated by actuator 112, the distal actuator 136, the internal bushing 134, the clevis 138, the link 156, and the pairs of jaws 160 are rotated, as well.

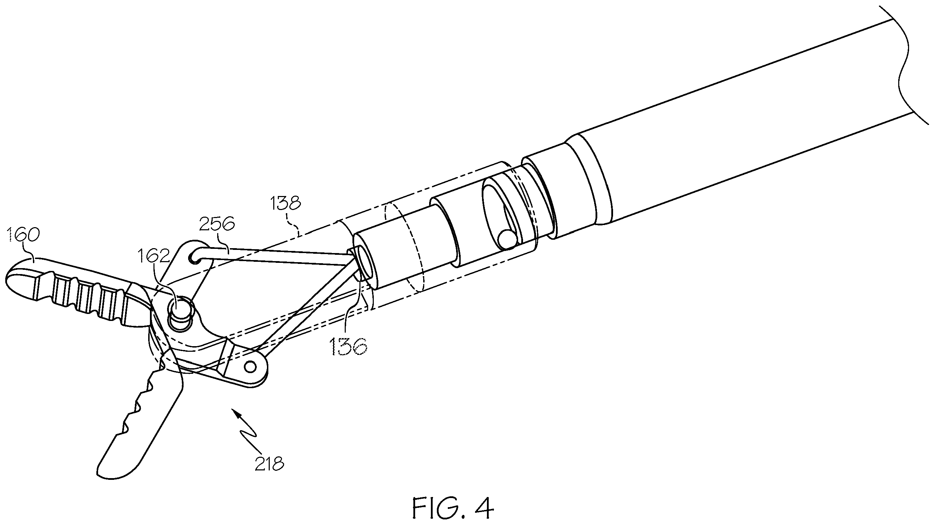

FIG. 4 is a perspective view of an alternate embodiment of a distal end effector assembly 218, with clevis 138 transparent (as represented by dashed lines) so that internal components are visible. Distal end effector assembly 218 is similarly configured to distal end effector assembly 118, shown in FIGs. 3A and 3B. However, a wire 256 replaces each link 156 and its associated pin 158 as shown in FIGs. 3A and 3B. Each wire 256 is fixed at a distal end to a proximal end of an associated jaw 160 and at a proximal end to distal actuator 136 via any suitable connection method. The wires 256 alternatively may extend through the entire length of shaft 116 to the handle 122, or the one or more wires 256 may converge to form a single wire within the distal end effector assembly that then either connects to actuation member or extends through shaft 116 to handle 122.

Aspects of the disclosure include methods of using device 100. To do so, the user may first introduce the distal end 120 of device 100 into a GI tract via a natural orifice. The orifice can be, for example, the nose, mouth, or anus, and the placement can be in any portion of the GI tract, including the esophagus, stomach, duodenum, large intestine, or small intestine. Delivery and placement also can be in other body lumens or organs reachable via the GI tract, any other natural opening or body tract, bodily incision, or through a delivery device, such as an endoscope or sheath. Once the desired site is accessed, the user can actuate one or more actuators, including actuators 108 and 112, to control the actuation and/or the rotation of the distal end effector assembly 118 relative to the shaft 116 of the medical device 100. The distal end effector 218 of FIG. 4 may be used and mechanized by the same or similar components as previously described.

Other embodiments of the disclosure will be apparent to those skilled in the art from consideration of the specification and practice of the invention disclosed herein. It is intended that the specification and examples be considered as exemplary only, with a true scope of the invention being indicated by the following claims.

Accordingly, various aspects discussed herein may help to improve the efficacy of treatment, for example, a procedure to treat a treatment site. Various aspects discussed herein may help to reduce and/or minimize the duration of the procedure, may reduce the risks of inadvertent manipulation by the user, and/or may help reduce risks of inadvertent contact with tissue or other material during delivery, repositioning, or usage of a medical device in the procedure.

While principles of this disclosure are described herein with reference to illustrative aspects for various applications, it should be understood that the disclosure is not limited thereto. Those having ordinary skill in the art and access to the teachings provided herein will recognize additional modifications, applications, aspects, and substitution of equivalents all fall within the scope of the aspects described herein. Accordingly, the disclosure is not to be considered as limited by the foregoing description.

## Claims

1. A distal end assembly of a medical device (100), comprising:
a clevis (138);
a bushing (134) at a proximal end (106) of the clevis (138);
an end effector (118, 218) pivotally connected to a distal end (120) of the clevis (138);
a distal actuator (136) connected to the end effector (118, 218), wherein translation of the distal actuator (136) actuates the end effector (118, 218); and
a shaft connector (132), wherein the bushing (134) is within the shaft connector (132) and rotatable relative to the shaft connector (132),
wherein the bushing (134) is moveably coupled to the distal actuator (136),
**characterised in that**
the bushing (134) is moveably coupled to the distal actuator (136) by a matching of a similarly shaped, non-round thru-hole (150) of the bushing (134) and the distal actuator (136).

2. The distal end assembly of claim 1, wherein the bushing (134) is fixed to the clevis (138), and/or wherein the bushing (134) is within the clevis (138).

3. The distal end assembly of any one of the preceding claims, wherein the end effector (118, 218) comprises an electrocautery grasper having two jaws.

4. The distal end assembly of any one of the preceding claims, wherein translation of the distal actuator (136) in a first direction opens the end effector (118, 218) and translation of the distal actuator (136) in a second direction closes the end effector (118, 218).

5. The distal end assembly of any one of the preceding claims, wherein the end effector (118, 218) is pivotably connected to the distal actuator (136) by one or more pins and one or more links.

6. The distal end assembly of any one of the preceding claims, wherein the clevis (138) is rotatable relative to the shaft connector (132).

7. The distal end assembly of any one of the preceding claims, wherein rotation of the distal actuator (136) causes rotation of the clevis (138) and the end effector (118, 218).

8. The distal end assembly of any one of the preceding claims, wherein a shoulder (144) of the shaft connector (132) and a flange (140) of the bushing (134) inhibit axial movement of the distal end assembly.

9. The distal end assembly of any one of the preceding claims, wherein the distal actuator translates within the bushing (134).

10. The distal end assembly of any one of the preceding claims, wherein the end effector (118, 218) is pivotably coupled to the distal actuator (136) by one or more wires.

11. The distal end assembly of any one of the preceding claims, wherein an annular gap is defined between the bushing (134) and the shaft connector (132).

12. The distal end assembly of any one of the preceding claims, wherein the shaft connector (132) defines a lumen extending therein and an opening at a distal end (120), the bushing (134) extending within the lumen and out of the opening.

13. The distal end assembly of any one of the preceding claims, wherein an annular gap is defined between the distal actuator (136) and the bushing (134).

14. The distal end assembly of any one of the preceding claims, wherein the bushing (134) defines a lumen therethrough, and the distal actuator (136) extends through the lumen.

## Patentansprüche

1. Distale Endanordnung einer medizinischen Vorrichtung (100), aufweisend:
einen Bügel (138);
eine Muffe (134) an einem proximalen Ende (106) des Bügels (138);
einen Endeffektor (118, 218), der mit einem distalen Ende (120) des Bügels (138) drehbar verbunden ist;
einen distalen Aktuator (136), der mit dem Endeffektor (118, 218) verbunden ist, wobei Translation des distalen Aktuators (136) den Endeffektor (118, 218) betätigt; und
einen Schaftverbinder (132), wobei die Muffe (134) in dem Schaftverbinder (132) ist und relativ zu dem Schaftverbinder (132) rotierbar ist,
wobei die Muffe (134) beweglich mit dem distalen Aktuator (136) gekoppelt ist,
**dadurch gekennzeichnet, dass**
die Muffe (134) mittels eines Zusammenpassens eines ähnlich gestalteten nichtrunden Durchgangslochs (150) der Muffe (134) und des distalen Aktuators (136) beweglich mit dem distalen Aktuator (136) gekoppelt ist.

2. Distale Endanordnung nach Anspruch 1, wobei die Muffe (134) an dem Bügel (138) befestigt ist, und/oder wobei die Muffe (134) innerhalb des Bügels (138) ist.

3. Distale Endanordnung nach einem der vorstehenden Ansprüche, wobei der Endeffektor (118, 218) einen Elektrokaustik-Greifer mit zwei Backen aufweist.

4. Distale Endanordnung nach einem der vorstehenden Ansprüche, wobei Translation des distalen Aktuators (136) in eine erste Richtung den Endeffektor (118, 218) öffnet, und Translation des distalen Aktuators (136) in eine zweite Richtung den Endeffektor (118, 218) schließt.

5. Distale Endanordnung nach einem der vorstehenden Ansprüche, wobei der Endeffektor (118, 218) mittels eines oder mehrerer Zapfen und eines oder mehrerer Bindeglieder drehbar mit dem distalen Aktuator (136) verbunden ist.

6. Distale Endanordnung nach einem der vorstehenden Ansprüche, wobei der Bügel (138) relativ zu dem Schaftverbinder (132) rotierbar ist.

7. Distale Endanordnung nach einem der vorstehenden Ansprüche, wobei Rotation des distalen Aktuators (136) eine Rotation des Bügels (138) und des Endeffektors (118, 218) bewirkt.

8. Distale Endanordnung nach einem der vorstehenden Ansprüche, wobei eine Schulter (144) des Schaftverbinders (132) und ein Flansch (140) der Muffe (134) eine axiale Bewegung der distalen Endanordnung verhindern.

9. Distale Endanordnung nach einem der vorstehenden Ansprüche, wobei der distale Aktuator in der Muffe (134) translatiert.

10. Distale Endanordnung nach einem der vorstehenden Ansprüche, wobei der Endeffektor (118, 218) mittels eines oder mehrerer Drähte drehbar mit dem distalen Aktuator (136) gekoppelt ist.

11. Distale Endanordnung nach einem der vorstehenden Ansprüche, wobei ein ringförmiger Spalt zwischen der Muffe (134) und dem Schaftverbinder (132) definiert ist.

12. Distale Endanordnung nach einem der vorstehenden Ansprüche, wobei der Schaftverbinder (132) ein sich darin erstreckendes Lumen und eine Öffnung an einem distalen Ende (120) definiert, wobei die Muffe (134) sich in dem Lumen und aus der Öffnung heraus erstreckt.

13. Distale Endanordnung nach einem der vorstehenden Ansprüche, wobei ein ringförmiger Spalt zwischen dem distalen Aktuator (136) und der Muffe (134) definiert ist.

14. Distale Endanordnung nach einem der vorstehenden Ansprüche, wobei die Muffe (134) ein hindurchgehendes Lumen definiert, und der distale Aktuator (136) sich durch das Lumen erstreckt.

## Revendications

1. Ensemble d'extrémité distale d'un dispositif médical (100) comprenant :
une chape (138) ;
une bague (134) au niveau d'une extrémité proximale (106) de la chape (138) ;
un effecteur terminal (118, 218) raccordé, de manière pivotante, à une extrémité distale (120) de la chape (138) ;
un actionneur distal (136) raccordé à l'effecteur terminal (118, 218), dans lequel la translation de l'actionneur distal (136) actionne l'effecteur terminal (118, 218) ; et
un connecteur de tige (132), dans lequel la bague (134) se trouve à l'intérieur du connecteur de tige (132) et peut tourner par rapport au connecteur de tige (132),
dans lequel la bague (134) est couplée, de manière mobile, à l'actionneur distal (136),
**caractérisé en ce que** :
la bague (134) est couplée, de manière mobile, à l'actionneur distal (136) par l'emboîtement d'un trou débouchant non circulaire de forme similaire (150) de la bague (134) avec l'actionneur distal (136).

2. Ensemble d'extrémité distale selon la revendication 1, dans lequel la bague (134) est fixée à la chape (138), et/ou dans lequel la bague (134) se trouve à l'intérieur de la chape (138).

3. Ensemble d'extrémité distale selon l'une quelconque des revendications précédentes, dans lequel l'effecteur terminal (118, 218) comprend une pince d'électrocoagulation ayant deux mâchoires.

4. Ensemble d'extrémité distale selon l'une quelconque des revendications précédentes, dans lequel la translation de l'actionneur distal (136) dans une première direction ouvre l'effecteur terminal (118, 218) et la translation de l'actionneur distal (136) dans une deuxième direction ferme l'effecteur terminal (118, 218).

5. Ensemble d'extrémité distale selon l'une quelconque des revendications précédentes, dans lequel l'effecteur terminal (118, 218) est raccordé, de manière pivotante, à l'actionneur distal (136) par une ou plusieurs broches et une ou plusieurs liaisons.

6. Ensemble d'extrémité distale selon l'une quelconque des revendications précédentes, dans lequel la chape (138) peut tourner par rapport au connecteur de tige (132).

7. Ensemble d'extrémité distale selon l'une quelconque des revendications précédentes, dans lequel la rotation de l'actionneur distal (136) provoque une rotation de la chape (138) et de l'effecteur terminal (118, 218).

8. Ensemble d'extrémité distale selon l'une quelconque des revendications précédentes, dans lequel un épaulement (144) du connecteur de tige (132) et une bride (140) de la bague (134) empêchent le mouvement axial de l'ensemble d'extrémité distale.

9. Ensemble d'extrémité distale selon l'une quelconque des revendications précédentes, dans lequel l'actionneur distal effectue une translation à l'intérieur de la bague (134).

10. Ensemble d'extrémité distale selon l'une quelconque des revendications précédentes, dans lequel l'effecteur terminal (118, 218) est couplé, de manière pivotante, à l'actionneur distal (136) par un ou plusieurs fils.

11. Ensemble d'extrémité distale selon l'une quelconque des revendications précédentes, dans lequel un interstice annulaire est défini entre la bague (134) et le connecteur de tige (132).

12. Ensemble d'extrémité distale selon l'une quelconque des revendications précédentes, dans lequel le connecteur de tige (132) définit une lumière s'étendant dans ce dernier et une ouverture au niveau d'une extrémité distale (120), la bague (134) s'étendant à l'intérieur de la lumière et à l'extérieur de l'ouverture.

13. Ensemble d'extrémité distale selon l'une quelconque des revendications précédentes, dans lequel un interstice annulaire est défini entre l'actionneur distal (136) et la bague (134).

14. Ensemble d'extrémité distale selon l'une quelconque des revendications précédentes, dans lequel la bague (134) définit une lumière à travers cette dernière, et l'actionneur distal (136) s'étend à travers la lumière.
